# EUROPEAN PATENT SPECIFICATION

(11) **EP 2 576 780 B1**
(45) Date of publication and mention of the grant of the patent: **25.02.2015**
(21) Application number: 11738288.7
(22) Date of filing: 30.05.2011
(51) Int. Cl.: C12N 15/10, C12Q 1/68, C40B 40/08, C40B 50/06

(54) **METHOD FOR THE PREPARATION AND AMPLIFICATION OF REPRESENTATIVE AND STRAND- SPECIFIC LIBRARIES OF CDNA FOR HIGH THROUGHPUT SEQUENCING, USE THEREOF, KIT AND CARTRIDGES FOR AUTOMATION KIT**
VERFAHREN ZUR HERSTELLUNG UND AMPLIFIKATION VON REPRÄSENTATIVEN UND STAMMSPEZIFISCHEN CDNA-BIBLIOTHEKEN FÜR HOCHDURCHSATZSEQUENZIERUNG, IHRE VERWENDUNG, KIT UND PATRONEN FÜR AUTOMATISIERUNGSKITS
PROCÉDÉ DE PRÉPARATION ET D'AMPLIFICATION DE BIBLIOTHÈQUES REPRÉSENTATIVES DE L'ADNc ET SPÉCIFIQUES À UN BRIN D'ADNc POUR UN SÉQUENÇAGE À HAUT RENDEMENT, LEUR UTILISATION, TROUSSE CORRESPONDANTE ET CARTOUCHES DESTINÉES À UNE TROUSSE D'AUTOMATISATION

(30) Priority: 31.05.2010 IT RM20100293
(43) Date of publication of application: 10.04.2013
(73) Proprietor: Consiglio Nazionale Delle Ricerche, 00185 Roma (IT)
(72) Inventor: TULLO, Apollonia, 00185 Roma (IT); MANGIULLI, Marina, 00185 Roma (IT); SBISA', Elisabetta, 00185 Roma (IT); PESOLE, Graziano, 00185 Roma (IT)
(74) Representative: Capasso, Olga
(86) International application number: PCT/IB2011/052369
(87) International publication number: WO 2011/151777

(56) References cited:
- WO-A2-02/057447
- WO-A2-2004/070053
- WO-A2-2006/086668
- WO-A2-2006/110314
- WO-A2-2007/019444
- DE-C1- 19 913 934
- US-A- 5 104 792
- US-A1- 2003 175 709
- US-B1- 6 613 516
- DEAN F B ET AL: "Rapid amplification of plasmid and phage DNA using Phi29 DNA polymerase and multiply-primed rolling circle amplification", GENOME RESEARCH, COLD SPRING HARBOR LABORATORY PRESS, WOODBURY, NY, US, vol. 11, no. 6, 1 June 2001 (2001-06-01), pages 1095-1099, XP002223174, ISSN: 1088-9051, DOI: DOI:10.1101/GR.180501
- "RiboMinusTM Transcriptome Isolation Kits" In: "New Product Catalog 2006", 2006, INVITROGEN, US, XP002614727, pages 33-34, the whole document

## Description

### FIELD OF THE INVENTION

The present invention relates to a rapid, simple, effective and economical method for preparing and amplifying representative and strand-specific cDNA libraries for next-generation high throughput sequencing, kit and cartridges for automation kit which use such method manually or in an automatic manner.

### PRIOR ART

Over the past few years new next-generation sequencing (NGS) platforms have been developed, including the "454 GS FLX" by Roche, "SOLiD" by Applied Biosystems and "Genome Analyzer" by Illumina. Initially used almost exclusively for sequencing and/or resequencing whole genomes and for determining single nucleotide polymorphisms (SNP), today they are increasingly employed in an effective manner for many other applications, including identification of biological markers and pathogenic agents in biodiversity studies and metagenomics in the biomedical, agri-food, environmental and industrial sectors. Among the most recent and promising applications, the sequencing of whole transcriptome is becoming fundamental for studying the regulation of gene expression. In fact, this type of analysis enables the identification and quantification not only of mRNAs, but also of small RNAs, such as micro-RNAs and other non-coding RNAs, whose biological meaning is still largely unknown. Among next-generation sequencing platforms, the Roche 454 GS FLX pyrosequencer makes it possible to obtain in each run over a million reads, each with a length of about 400 bases, even up to about 800 bases. In addition to enabling the identification and relative quantification of mRNAs, sequences of this length also permit the identification of new splicing sites and variants, expressed in different physiopathological conditions. The shorter length of the reads, but of higher number, of the platforms produced by Illumina (100-150 bp) and Applied Biosystems (35-75 bp), on the other hand, makes them particularly suitable for sequencing small RNAs and quantifying the levels of transcripts.

Irrespective of the NGS platform used, the first step required for transcriptome analysis is the construction of cDNA libraries for high throughput sequencing.

One of the main problems is represented by the fact that the starting amount of RNA is often very exiguous and that some transcripts, despite having considerable biological significance, are very scarcely abundant. To this it may be added that around 90-95% of the total RNA extracted consists of ribosomal RNA (rRNA) which, for a successful experiment, should be removed. This means starting off with a higher amount of total RNA, which is not always available.

Existing protocols for cDNA library construction involve the following steps: RNA retrotranscription, synthesis of the second strand and in some cases amplification of the retrotranscribed RNA species. MRNA retrotranscription can be done using oligo-dT or small random primers. In general it is preferred to use oligo-dT because rRNAs are not polyadenylated and as a result are excluded in the retrotranscription reaction. The use of oligo-dT has the large disadvantage of excluding not only rRNAs from the retrotranscription reaction, but also every non-polyadenylated species of RNA, such as the RNA species with a regulatory function or bacterial or viral RNA species (metatranscriptomics) that it is desirable to identify in human cells in some physiological or pathological conditions, such as, for example, in the case of multiple sclerosis. Moreover, the long T or A homopolymer tails can give rise to sequences of poor quality, in particular for the Roche 454 GS FLX sequencer. In order to remedy this technical problem, it is possible to use oligo-dT primers with a sequence at 5' containing a restriction site, which cleaves 14-16 nt downstream. In this manner the T tail can be removed after retrotranscription; otherwise, it is possible to use for retrotranscription oligo-dT primers in which another nucleotide is inserted in every 4 or 5 T, so as to interrupt the long T tail. The synthesis of the second strand complementary to the retrotranscribed strand is in some cases followed by an amplification reaction using adapters, which are ligated to the ends of the retrotranscribed fragments. This makes it possible to amplify the starting material and in particular the least represented species of RNA which are otherwise hardly detected. For the amplification reaction, Taq polymerase is generally used. However, this protocol has the drawback of producing a population of fragments smaller than 2Kb; as a result, nebulization, which is the fragmentation process used in the standard procedure for preparing shotgun libraries for sequencing, might not give optimal results, since it is more suitable for molecules with a high molecular weight.

Alternative protocols provide for the isolation of mRNAs with oligo-dT, fragmentation of the RNA molecules, gel purification of low molecular weights and then retrotranscription. In this case the nebulization step is omitted. Amplification of the retrotranscribed fragments is not envisaged, with the drawback that the least represented RNA species cannot be identified.

At the same time, another important element for the preparation of cDNA libraries for massive sequencing is the preservation of the information relating to which of the two DNA strands was actually transcribed and thus belongs to the original pool of mRNA; this characteristic is hereinafter called strand specificity. In recent years it has been seen that many genomic regions give rise to transcripts originating from both DNA strands. Antisense transcription plays an important regulatory role. Moreover, overlapping genes are common in the compact genomes of prokaryotes and lower eukaryotes.

It follows that distinguishing from which DNA strand each RNA has been transcribed - RNA that will then be retrotranscribed into cDNA in a library - considerably increases the value of a transcriptome experiment because, during the mapping of sequences on the reference genome, it renders unambiguous the attribution of reads to the DNA strand from which the RNA was transcribed. Therefore, information about strand specificity makes it possible to distinguish coding transcripts from antisense ones, to determine from which strand other non-coding RNAs are synthesized, to define more precisely the exact boundaries of adjacent genes, transcribed from opposite strands, and to determine the correct levels of expression of coding and non-coding RNA molecules, transcribed from overlapping sequences.

WO2006/086668 describes a method for obtaining a representative library in which the possibility of verifying the strand specificity of the cDNAs contained therein is not guaranteed. The primers of the invention are not described.

WO2004/070053 also describes a method for obtaining a representative library in which the possibility of verifying the strand specificity of the cDNAs contained therein is not guaranteed. In that document as well, the primers of the invention are not described.

Disclosed in the state of the art are numerous methods for preserving information about the strand that fall into two groups: one involves the ligation of distinct adapters, with known orientation, at 5' and 3' of the transcript, prior to retrotranscription, whereas in the other one, a strand is marked by introducing chemical modifications allowing it to be distinguished from the complementary strand. Therefore, it doesn't exist a method for preparing representative and strand-specifc cDNA libraries which solves the problems of the prior art.

### DESCRIPTION OF THE INVENTION

The present invention enables the preparation of cDNA libraries which can be sequenced by means of the principal massive sequencing platforms today available on the market. The method of the invention solves the described problems providing representative and strand-specific libraries in a rapid, simple and economical manner.

The innovative aspects of the procedure regard the study of the order of the various steps for preparing the sample and optimization of the performance of massive sequencing. In particular the new process is able to work with:
1. use of exiguous starting amounts of total RNA (even 400-500 ng);
2. considerable reduction in rRNA (about 90%);
3. retrotranscription starting from 30-40 ng of polyadenylated and non-polyadenylated transcripts with specific primers capable of preserving strand information, designed for the purpose;
4. ligation of the retrotranscripts;
5. amplification with DNA polymerase with high fidelity and processivity, such as for example Phi29;
6. high yields of amplified DNA (from around 30 µg).

With the strategy devised it is possible to subject to massive sequencing even samples of precious RNAs available in small amounts and otherwise hardly analysable, above all as regards poorly represented transcripts. The fact that the starting amounts of RNA (both total and devoid of rRNA) are halved compared to many protocols and that with the high yields achieved through the procedure of the invention it is possible to use the cDNA library for further subsequent validation experiments, and the strand specificity, are also extremely relevant.

The innovative aspects of the procedure regard the definition of the order of the various steps for preparing the sample and optimization of the performance of massive sequencing both for the production of data and for analysis.

In this context the authors have designed a rapid, simple, effective, economical procedure which makes it possible to start off with halved amounts of total RNA (400-500 ng), to considerably reduce rRNA (about 90%), to construct a library of polyadenylated and non-polyadenylated transcripts that is also representative of the least abundant species as it includes an amplification step after retrotranscription, and to preserve strand specificity, which, at the time of analysis, allows to identify which DNA strand was actually transcribed.

A cDNA library represents the set of transcripts expressed by a specific cellular system. Highly expressed genes will be represented by many transcripts in the library, whereas genes expressed at low levels will be less represented, or even not at all. A cDNA library is said to be representative when it contains, in at least one copy, all of the transcripts, including the least abundant ones. A cDNA library is said to be strand specific when it enables identification of the DNA strand from which the RNA, later retrotranscribed to cDNA, was transcribed.

DNA polymerases differ from one another in a number of properties, such as processivity, proofreading activity, strand displacement activity, i.e. the capacity to displace the DNA encountered downstream during synthesis, and yields. Among the available DNA polymerases, the following table indicates which have high levels of proofreading and strand displacement activity, and the associated error rate.

| | **3'→5' Exonuclease** | **Error rate (x10⁻⁶)** | **Strand Displacement** |
|---|---|---|---|
| *Bst* DNA Polymerase, Large Fragment | - | | ++++ |
| *Bst* DNA Polymerase, Full Length | - | | - |
| *Taq* DNA Polymerase | - | 285 | - |
| Vent_{R}® DNA Polymerase | ++ | 57 | ++ |
| Vent_{R}® (exo-) DNA Polymerase | - | 190 | +++ |
| Deep Vent_{R}® DNA Polymerase | +++ | | ++ |
| Deep Vent_{R}® (exo-) DNA Polymerase | - | | +++ |
| 9°Nₘ™ DNA Polymerase | + | | +++ |
| T7 DNA Polymerase | ++++ | 15 | - |
| *E. coli* DNA Polymerase I | ++ | 9 | - |
| T4 DNA Polymerase | ++++ | <1 | - |
| phi29 DNA Polymerase | ++++ | 9,5 | +++++ |
| Phusion® High-Fidelity DNA Polymerase | ++++ | 0.44 | |
| Phusion® High-Fidelity Hot Start DNA Polymerase | ++++ | 0.44 | |
| Phusion® High-Fidelity Hot Start II DNA Polymerase | ++++ | 0.44 | |
| Phire® Hot Start DNA Polymerase | + | | - |
| Phire® Hot Start II DNA Polymerase | + | | - |
| Phusion® Flash High-Fidelity DNA Polymerase | ++++ | | |
| LongAmp™ *Taq* DNA Polymerase | ++ | | - |

The authors have used various DNA polymerases, including Phi29 Polymerase, which are distinguished by the following characteristics: i) strand displacement activity, which enables effective and uniform amplification also in the presence of secondary structures and sequences hardly amplifiable; ii) proof-reading activity among the highest today available in the realm of high-fidelity Taq Polymerases; iii) high processivity, which allows the generation of very long synthesized products, ideal for the nebulization step, without disassociation from the template; iv) high yields of amplified product starting from small amounts of template. Analogous polymerases can likewise be used.

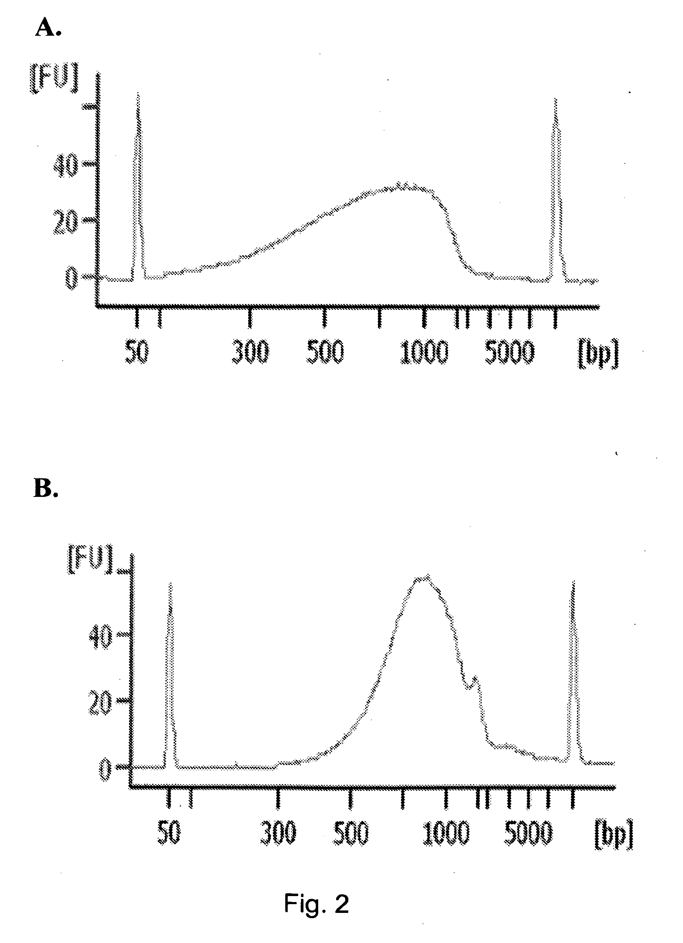

In fact, the method of the present invention makes it possible to obtain, starting from an exiguous amount of total RNA (about 400-500 ng), about 30-40 ng of RNA (the rRNA component considerably reduced) to be retrotranscribed and amplified with a final yield greater than about 30 µg of high molecular weight DNA. Therefore, the method is particularly suitable for achieving excellent fragmentation results with nebulization (see Figure 2). This makes it possible to have material that is more than sufficient not only for the massive sequencing procedure, but also for further analyses and validations subsequent to the result of sequencing, such as determination of the gene expression profile by Real Time PCR. In fact, the method of the invention enables a uniform amplification of all transcripts present in the starting RNA, thanks to the intrinsic properties of Phi29 polymerase and the entire amplification procedure. In the present invention it is possible to subject to massive sequencing and further validation even precious samples of RNA and available in small amounts, otherwise hardly analysable, especially as regards poorly represented transcripts. The present invention also enables to preserve information about strand specificity so as to be able to correctly determine the direction of transcription and explore the transcriptome complexity in a more effective and accurate manner.

In the present invention a method has been developed for the preparation of cDNA libraries for massive sequencing defining the order of protocols to be used. Therefore, object of the invention is a method for obtaining a representative and strand-specific cDNA library from an RNA sample of at least about 400 ng, comprising the steps of:
a) removing ribosomal RNA from the total RNA extracted in order to obtain a fraction of rRNA-depleted RNA in which said fraction is present in a amount of about at least 30 ng;
b) retrotranscribing the rRNA-depleted fraction of RNA to cDNA;
c) purifying the synthesized cDNAs;
d) ligating and amplifying the purified cDNA to obtain a cDNA library of at least about 30 µg, in which the retrotranscription step is performed in the presence of 5' phosphorylated primers having one of the following sequences:
   5'-TCGCGATCGTCGNNNNNNNN-3' (SEQ ID No. 2) or 5'-GCGGCCGCNNNNNN-3' (SEQ ID No. 1),
   where N is any one of the four nucleotides A, T, G and C, and the primers are present in equimolar amounts, and wherein the amplification step is performed in the presence of Phi29 DNA polymerase with high strand-displacement and proof-reading activity, high processivity and yields.

In a preferred aspect the method of the invention also comprises a preliminary step of extracting total RNA from a sample, for example a biological, cellular, etc. sample.

A person skilled in the art can select other DNA polymerases with activity comparable to that of the DNA polymerase Phi 29. In a preferred aspect, the primers are present in an amount of about 25 to about 50 µM and the rRNA-depleted RNA is present in an amount of about 30 ng to about 300 ng. In a more preferred aspect the primers are present in an amount of about 50 µM. A further object of the invention is the use of the representative and strand-specific cDNA library as above defined for high throughput sequencing.

In consideration of the high sample sequencing processivity of NGS platforms, the possibility of preparing the libraries in an automated manner constitutes an advantage in terms of time and number of samples. For this purpose the reaction components are prepared in cartridges, i.e. supports containing in micro-containers, in an orderly and sequential manner, all or part of the reagents (solutions, primers etc.) in the amount and order envisaged by the method. The cartridges are advantageously inserted in automation platforms (liquid handlers) which run the reactions of the protocol (or some steps thereof) in an automated and controlled manner (controls on the precision of the aspirated volumes, aerosols, sterility, etc.).

The method of the invention, by means of cartridges usable in an automated or semi-automated system, is capable of:
- handling a large number of samples (also tagged with barcodes)
- optimizing sample preparation times
- eliminating any volume errors and aerosol
- optimizing the amount of the numerous reagents included in the kit to be used manually
- simplifying its execution with a single product.

The present invention will be described through non-limitative examples, with reference to the following figures:
Figure 1. Agilent profile of test RNA subjected to the "RiboMinus Eukaryote Kit for RNA Seq" (Invitrogen) (in blue) compared with the profile of the respective total starting RNA (in red). Note how the peaks corresponding to 28S and 18S ribosomal RNAs, evident in the starting sample, are nearly absent in the post-Ribominus sample, which is thus enriched in the non-ribosomal component of RNA.
Figure 2. Agilent profile of: A. a library after nebulization and B. after removal of low molecular weights. Both profiles correspond to the conditions recommended for preparing shotgun libraries to be subjected to sequencing with the 454 GS FLX.
Figure 3. Results of sequencing, conducted with the Roche 454 GS FLX Titanium platform, of two transcriptome samples prepared with the primer of SEQ ID No. 1.
Figure 4. Results of sequencing, conducted with the Roche 454 GS FLX Titanium platform, of two transcriptome samples prepared with the primer of SEQ ID No. 2.

### MATERIALS AND METHODS

### Extraction of total RNA

The total RNA was extracted from cell lines and tissues using the *RNeasy plus mini* kit (QIAGEN Cat. No. 74134), which assures efficient removal of genomic DNA and enrichment in transcripts more than 200 nucleotides long (a large part of the RNAs shorter than this length are selectively removed).

### Removal of ribosomal RNA (rRNA)

The removal of the ribosomal component from the total starting RNA was effected using the *RiboMinus Eukaryote Kit for RNA Seq* (Invitrogen Cat. No. A1083708).

rRNA removal was conducted according to the procedure indicated by the manufacturer, shown here below:
1. Set a thermostatically-controlled water bath to 75°C.
2. To a sterile 1.5 ml tube add:

| Component | Volume per reaction |
|---|---|
| Total RNA | minimum 400 ng (*) in <20µl |
| Ribominus probe (15pmol/µl) | 10µl |
| Hybridization Buffer | 300µl |

| | |
|---|---|
| (*) Though the kit indicates 2µg as the minimum amount of starting material, the authors have verified that, starting from 400-500 ng of total RNA, it is possible obtain around 30-40 ng of RNA depleted of the ribosomal fraction, sufficient for the construction and amplification of a representative and strand-specific cDNA library prepared according to the procedure of the present invention. | |

3. Incubate the tube at 75°C for 5 minutes to denature RNA.
4. Allow the sample to cool slowly and incubate at 37°C for 30 min. To promote sequence-specific hybridization between rRNA and *Ribominus probes* (eukaryote rRNA specific and 5'-biotin labelled probes) it is important to allow slow cooling.
While the sample is at 37°C, prepare the streptavidin-coated magnetic beads.
5. Resuspend the solution containing the beads by vortexing.
6. Pipet 750µl of the bead suspension into a sterile 1.5ml tube.
7. Place the tube on a magnetic separator for 1 min, waiting for the beads to settle on the tube side closest to the magnet, aspirate and discard the supernatant.
8. Add 750µl of H₂O-DEPC to the beads and resuspend them by gentle vortexing.
9. Again place the tube on the magnetic separator for 1 min, aspirate and discard the supernatant.
10. Repeat steps 8 and 9 once.
11. Resuspend the beads in 750µl of Hybridization Buffer and transfer 250µl of beads to a new tube.
12. Place the tube with the remaining 500µl of beads on the magnetic separator for 1 min, aspirate and discard the supernatant.
13. Resuspend the bead pellet in 200µl of Hybridization Buffer and maintain the tube at 37°C until use.
14. After the incubation at 37°C for 30 minutes of the hybridized sample, briefly centrifuge to collect it at the bottom of the tube.
15. Transfer the sample (about 330µl) to the previously prepared beads (step 13) and mix well.
16. Incubate in the water bath at 37°C for 15 min. During incubation, gently mix about every 2 minutes. At the end, briefly centrifuge to collect the sample at the bottom of the tube.
17. Place the tube on the magnetic separator for 1 min to allow the rRNA-probe complex to adhere to the sides. Collect the about 530µl supernatant containing rRNA-depleted RNA.
18. Place the tube containing 250µl beads (from step 11) on the magnetic separator for 1 min, aspirate and discard the supernatant.
19. To this tube with beads attached to the side, add the about 530µl supernatant containing the RNA of interest (from step 17). Mix well. The RNA already depleted of rRNA is then incubated a second time with the streptavidin beads to assure better removal.
20. Incubate the tube at 37°C for 15 min. During incubation, gently mix about every 2 min. At the end, briefly centrifuge to collect the sample at the bottom of the tube.
21. Transfer the supernatant containing RNA depleted of the ribosomal component, about 530µl, to a new 2 ml tube.

The subsequent steps, necessary in order to purify and concentrate the isolated RNA, require the use of the *Ribominus Concentration Module* (Invitrogen Cat. No. K155005), used according to the manufacturer directions:
22. Add to the tube containing RNA depleted of the ribosomal component 1 volume of Binding Buffer and 1 volume of 100% Ethanol. Mix thoroughly.
23. Transfer 700µl of this mixture to the column supplied with the kit.
24. Centrifuge at ≥ 12000 x g for 1 min at room temperature. Discard the flow-through.
25. Repeat steps 23 and 24 until the entire sample (from step 22) is loaded in the column.
26. Add to the column 200µl of Wash Buffer complemented beforehand with 100% ethanol (6ml 100% ethanol per 1.5ml of Wash Buffer).
27. Centrifuge at ≥ 12000 x g for 1 min at room temperature. Discard the flow-through.
28. Repeat the wash with another 200µl of Wash Buffer complemented with 100% ethanol.
29. Discard the *Collection Tube* supplied with the kit and place the column into the *Wash Tube.*
30. Centrifuge the column at maximum speed for 2-3 min at room temperature to remove any residual Wash Buffer. Discard the *Wash Tube.*
31. Place the column in the *Recovery Tube* supplied with the kit.
32. Add 10-15µl (●) of RNase-free Water to the centre of the column. Incubate at room temperature for 1 min.
33. Centrifuge at maximum speed for 1 min at room temperature.
34. The *Recovery Tube* now contains purified, concentrated RNA depleted of the ribosomal component. Place immediately on ice to proceed with downstream applications or store at -80°C until use. This is the last step provided by the kit.
(●) in the present invention the sample was eluted in 15µl of RNase-free H₂O supplied with the kit.

The concentration of the eluted RNA was determined using a Thermo Scientific Nanodrop 2000c micro-volume spectrophotometer.

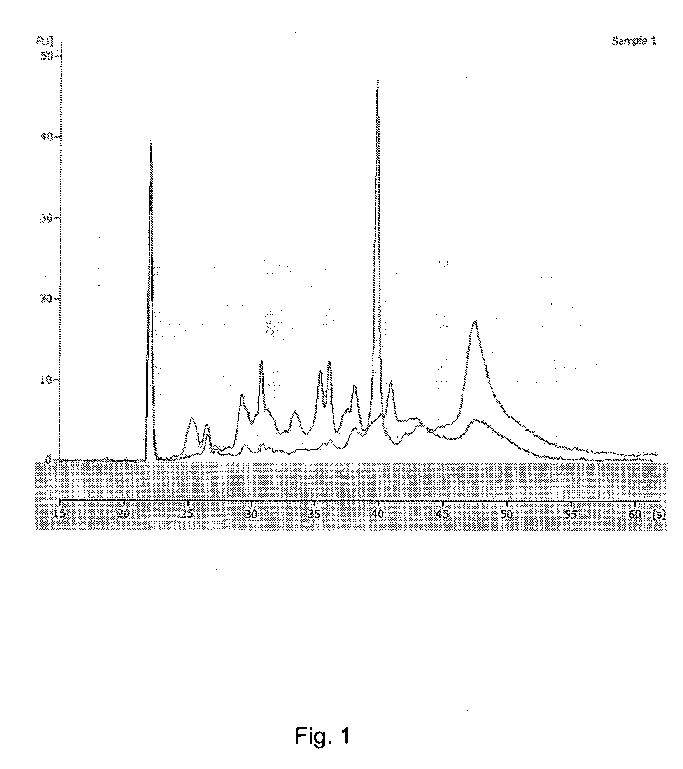

The efficiency of removal of ribosomal RNAs from the total starting RNA was evaluated by means of an Agilent 2100 bioanalyzer using the specific chip for RNA. As can be seen in Figure 1, where an example case of the results obtained is shown, the profile for the RNA subjected to the aforesaid procedure shows an efficient removal of 18S and 28S ribosomal RNAs relative to the total starting RNA.

### Retrotranscription

The RNA depleted of the ribosomal component was converted into single-stranded cDNA using the "SuperScript III (SSIII) First-strand synthesis system for RT-PCR" (Invitrogen Cat. No. 18080-051).

The associated protocol was modified in relation to some components, times and reaction conditions. Of extreme importance was the use of specific primers suitably designed by the authors so as to render applicable the cDNA amplification strategy, envisaged by the "Quantitect Whole Transcriptome" kit, and included in the present invention as step 5' in the procedure described herein, to the sample preparation for the principal ultra massive sequencing technologies available today. The authors also fine tuned the concentration of such primers in the retrotranscription reaction.

The RNA retrotranscription was performed according to the following protocol defined by the authors, who substituted the first step - single-stranded cDNA synthesis - of the "Quantitect Whole Transcriptome" kit (QIAGEN Cat. No. 207043):
1'. Prepare Mix 1 in a 0.5ml Eppendorf tube, adding the components listed here below:

| Component | Amount/Volume per reaction |
|---|---|
| post-Ribominus RNA (Fig. 1) | minimum about 30 ng (**) |
| *Tag-Lig-hexamers or octamers* (50µM) | 1µl (***) |
| dNTPs (10mM) | 1µl |
| H₂O-DEPC | up to 10µl |

| | |
|---|---|
| (**) One could start off from an amount of RNA depleted of the ribosomal component smaller than 30 ng (even 1pg), but that would increase the risk that transcripts with a low number of copies might be only partially represented or absent from the sample at the start of the transcriptome amplification step. This in turn cannot assure high reproducibility in the cDNA amplification. However, an experimenter can assess whether to start off from a smaller amount of RNA based on his own experimental requirements and on the number of reads he intends to obtain for each transcript of the examined sample, in particular for the least represented ones. (***) The primers, indicated as *Tag-Lig-octamers or hexamers,* are 5'-phosphorylated and have sequences, respectively: 5'-TCGCGATCGTCGNNNNNNNN-3' (SEQ ID No. 2) or 5'-GCGGCCGCNNNNNN-3' (SEQ ID No. 1), where N represents any one of the four nucleotides. They are random octamers or hexamers preceded at 5' by a 12 or 8 bp sequence. In the primer of SEQ ID No. 2, the TCG trinucleotide precedes and follows the restriction site (underlined) of the rare-cutter enzyme PvuI, very rarely present in the human genome, at considerably lower frequencies than other restriction sites considered rare because present approximately once every 10 Mb. The trinucleotide upstream and downstream of the restriction site assures that strand information will be preserved, since its sequence, not being palindromic, unlike the PvuI site, is different depending on whether it is read in a 5'-3' direction on one DNA strand or on its complementary strand (palindromic sequence means a sequence of nucleic acid - DNA or RNA - which is the same whether read in a 5'-3' direction on one strand or its complementary strand). More precisely, the cDNA strand directly complementary to the mRNA from which it was synthesized will contain the sequence 5'-TCG-3' upstream and downstream of the PvuI site, whereas the strand complementary to it, and hence identical to the starting mRNA, will contain the trinucleotide 5'-CGA-3' in the same positions. Accordingly, following ligation of the massive sequencing adapters to the double-stranded cDNA, the information on strand specificity will not be lost. The trinucleotide 5'-TCG-3' was selected precisely because it was the rarest result in the RefSeq *Homo Sapiens* collection, representative of the human transcriptome, as well as the 12 bp sequence formed by the PvuI site preceded and followed by the trinucleotide 5'-TCG-3' resulted considerably rare. The necessity of inserting a known sequence at 5' is tied not only to maintaining strand information, but also to the nature of the amplification procedure used with the "Quantitect Whole Transcriptome" kit (QIAGEN Cat. No. 207043), which envisages a step of ligating all the single-stranded cDNA molecules to give concatenamers around 20kb long. The presence of the known sequence enables identification of the points where the ligation occurred. The fact that it is a particularly rare sequence makes it more functional as a label of the synthesized cDNAs. In the primer of SEQ ID No. 1 is present the restriction site for NotI, another rare cutter enzyme. | |

2'. Incubate Mix1 at 75°C for 5 min
3'. Place immediately on ice for 5 min.
4'. During this time interval, prepare Mix2, adding each component in the order indicated, with the modifications introduced by the authors:

| Component | Volume per reaction |
|---|---|
| 10X RT Buffer per SSIII | 2µl |
| MgCl₂ (25Mm) | 4µl |
| DTT (0.1M) | 2µl |
| RNaseOUT (40U/µl) | 1µl |
| SuperScript III reverse transcriptase (200U/µl) | 1µl |
| DMSO (SIGMA-Aldrich) | 0.5µl |
| Total volume | 10.5µl |

5'. Add Mix2 to Mix1, mix and collect at the bottom by brief centrifugation.
6'. Incubate the final mix thus obtained as indicated below with the authors' modifications:

| Temperature | Duration |
|---|---|
| 25°C | 10 min |
| 45°C | 90 min |
| 95°C | 5 min |

7'. After blocking the reaction with ice, add 1µl of RNaseH and incubate at 37°C for 20 min.
8'. The synthesized cDNA can be stored at -20°C or immediately used for the subsequent steps of the procedure.

### Purification of synthesized cDNAs

The cDNA synthesis reaction was subjected to purification using the *MinElute PCR Purification* kit (QIAGEN Cat. No. 28004) to eliminate primers, nucleotides, enzymes and salts, following the instructions provided in the kit and eluating the cDNA mixture in 10µl of Buffer EB (10mM Tris·Cl, pH 8.5).

### cDNA ligation and amplification

The purified cDNAs resuspended in Buffer EB (§) were ligated together in long concatenamers and subsequently amplified following the steps after the first retrotranscription step of the *QuantiTect Whole Transcriptome* kit (QIAGEN Cat. No. 207043) (see retrotranscription protocol).

(§) It was verified that Buffer EB does not interfere with the subsequent cDNA ligation and amplification procedures envisaged by the *QuantiTect Whole Transcriptome* kit (QIAGEN Cat. No. 207043).

The cDNA ligation reaction was carried out according to the following protocol envisaged by the aforesaid kit, except for the mixture of the purified retrotranscripts (step 2"):
1".Prepare the ligation Mix, adding the individual components in the following order:

| Component | Volume per reaction |
|---|---|
| Ligation Buffer | 6µl |
| Ligation Reagent | 2µl |
| Ligation Enzyme 1 | 1µl |
| Ligation Enzyme2 | 1µl |
| Total Volume | 10µl |

2".Add the 10µl of the ligation Mix to the 10µl of the purified cDNA mixture, mix and briefly centrifuge.
3".Incubate at 22°C for 2h,

After incubation:
4".Prepare the amplification Mix, which makes use of Phi29 *REPLI-g* polymerase (from the *QuantiTect Whole Transcriptome* kit, QIAGEN Cat. No. 207043), according to the following scheme:

| Component | Volume per reaction |
|---|---|
| REPLI-g Midi Reaction Buffer | 29µl |
| REPLI-g Midi DNA Polymerase | 1µl |
| Total volume | 30µl |

5 ".Add the 30µl of the amplification Mix to the 20µl of the ligation reaction, mix well and collect at the bottom by brief centrifugation
6".Incubate at 30°C for about 4 hours (or even for 2h, as indicated in the kit)
7".Stop the amplification reaction, incubating at 95°C for 5 min
8".Store the amplified cDNA at -20°C until subsequent use.

The cDNA libraries thus obtained, in the form of concatenamers about 20 kb long, were sequenced with excellent results following the standard protocols for the preparation of shotgun libraries for sequencing with the Roche 454 GS FLX platform.

### RESULTS

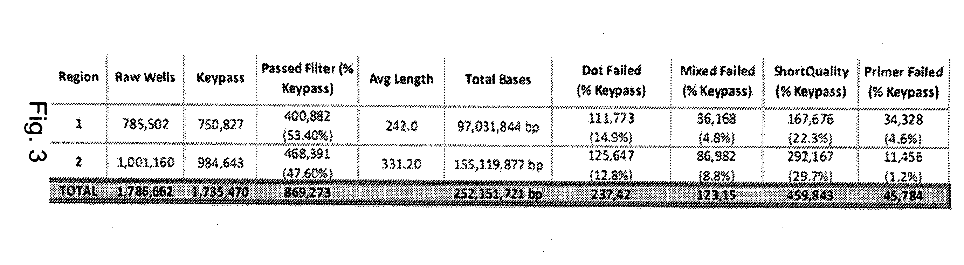

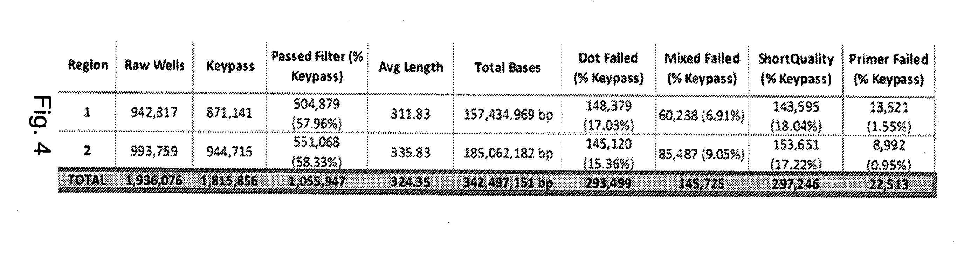

Examples of Agilent profiles for a representative and strand-specific library after nebulization of the amplified sample and after removal of low molecular weight fragments are shown in Figures 2A and 2B. The sequencing results with respect to two samples are presented in Figures 3 and 4. Following the method of the invention, a number of reads was obtained (see *passed filter* column) which was very close to the values corresponding to the highest performances of the 454 GS FLX pyrosequencer, indicated by the manufacturer.

The protocol developed in the present invention can be used to realize a new kit and/or cartridge for automation kits capable of assuring, in rapid times and at moderate costs, a uniform, faithful amplification of all transcripts in samples of small amounts of RNA to be retrotranscribed (30-40 ng) - depleted beforehand of the ribosomal component - thanks to the singular properties of Phi29 polymerase and information about strand specificity. At the same time the kit and/or cartridge for automation kits enables sequencing for transcriptome and metatranscriptome studies using the main NGS platforms (454 GS FLX by Roche, SOLiD by Applied Biosystems and Genome Analyzer by Illumina). Thanks to the high yields of amplified DNA (∼30 µg), moreover, it is possible to use the cDNA library produced for further subsequent validation experiments. Finally, the presence of the sequence at 5' of retrotranscription primers of SEQ ID No. 2, makes it possible to preserve information about the direction of transcription, rendering unambiguous the attribution of reads to the strand the RNA was transcribed from. An unambiguous attribution considerably simplifies the step of bioinformatic analysis downstream of the massive sequencing of the transcriptome. The kit may therefore represent an evolution of the "Quantitect Whole Transcriptome" (QIAGEN), since it contains the same last two steps of the protocol thereof, i.e. ligation of the single-stranded cDNAs and amplification of the concatenamers by Phi29 polymerase, while substituting the first retrotranscription step with the one described in the Materials and Methods section, paragraph on Retrotranscription, carried out with primers designed by the authors and in the conditions defined in the present invention, such as to make the amplified product suitable for subsequent ultra massive sequencing while at the same time maintaining information about the direction of transcription.

Considering the complexity of the procedures envisaged by NGS platforms and the high cost of a single run, the availability of a single and standardized kit for preparing transcriptome samples and capable of optimizing sequencing performance, the number of reads useful for analysis and information of strand-specificity is of extreme interest for users. It should be considered, in fact, that the number of NGS platforms present in the market is growing constantly and rapidly and in Europe alone there are currently: 200 Roche units, 210 Illumina units and 80 Applied Biosystems units, many of which in service.

## Claims

1. Method for obtaining a representative and strand-specific cDNA library from an RNA sample of at least about 400 ng, comprising the steps of:
a) removing the ribosomal RNA from the RNA to obtain an rRNA-depleted fraction of RNA wherein said fraction is present in an amount of at least 30 ng;
b) retrotranscribing the rRNA-depleted fraction of RNA to cDNA;
c) purifying the synthesized cDNA;
d) ligating and amplifying the purified cDNAs to obtain a cDNA library of at least about 30 µg,
wherein the retrotranscription step is performed in the presence of 5' phosphorylated primers having one of the following sequences:
5'-TCGCGATCGTCGNNNNNNNN-3' (SEQ ID No.2) or 5'-GCGGCCGCNNNNNN-3' (SEQ ID No. 1),
where N is any one of the four nucleotides A, T, G and C, and the primers are present in equimolar amounts, and wherein the amplification step is performed in the presence of Phi29 DNA polymerase with high strand-displacement and proof-reading activity, high processivity and yields.

2. Method according to claim 1, comprising a preliminary step of extracting the total RNA from a sample.

3. Method according to any of previous claims, wherein the primers are present in an amount of about 25 to about 50 µM and the rRNA-depleted RNA is present in an amount of about 30 ng to about 300 ng.

4. Method according to claim 3, wherein the primers are present in an amount of about 50 µM.

5. Use of the representative and strand-specific cDNA library according to any one of claims 1-4 for high throughput sequencing.

## Patentansprüche

1. Verfahren zum Erhalt einer repräsentativen und strangspezifischen cDNA-Bibliothek von einer RNA-Probe mit wenigstens etwa 400 ng, umfassend die folgenden Schritte:
a) Entfernen der ribosomalen RNA von der RNA, um eine rRNA-arme Fraktion von RNA zu erhalten, wobei die Fraktion in einer Menge von wenigstens 30 ng vorliegt.
b) Retrotranskribieren der rRNA-armen Fraktion der RNA zu cDNA;
c) Reinigen der synthetisierten cDNAs;
d) Ligieren und Amplifizieren der gereinigten cDNAs, um eine cDNA-Bibliothek von wenigstens etwa 30 µg zu erhalten,
wobei der Schritt der Retrotranskription in der Gegenwart von 5'-phosphorylierten Primern durchgeführt wird, die eine der folgenden Sequenzen aufweisen:
5'-TCGCGATCGTCGNNNNNNNN-3' (SEQ ID Nr. 2) oder 5'- GCGGCCGCNNNNNN-3' (SEQ ID Nr. 1.),
wobei N ein beliebiges der vier Nukleotide A, T, G und C ist, und die Primer in equimolaren Mengen vorliegen, und wobei der Schritt der Amplifizierung in der Gegenwart einer Phi29-DNA-Polymerase mit einer hohen strangverlagerungs- und korrekturleseaktivität, einer hohen Prozessivität und hohen Ausbeuten durchgeführt wird.

2. Verfahren nach Anspruch 1, umfassend einen Vorschritt des Extrahierens der Gesamt-RNA aus einer Probe.

3. Verfahren nach einem der vorangehenden Ansprüche, wobei die Primer in einer Menge von etwa 25 bis etwa 50 µM vorliegen, und die rRNA-arme RNA in einer Menge von etwa 30 ng bis etwa 300 ng vorliegt.

4. Verfahren nach Anspruch 3, wobei die Primer in einer Menge von etwa 50 µM vorliegen.

5. Verwendung der repräsentativen und strangspezifischen cDNA-Bibliothek nach einem der Ansprüche 1 bis 4 für eine Sequenzierung mit hohem Durchsatz.

## Revendications

1. Procédé destiné à obtenir une banque d'ADNc représentative et spécifique au brin à partir d'un échantillon d'ARN d'au moins environ 400 ng, comprenant les étapes suivantes :
a) extraction de l'ARN ribosomique à partir de l'ARN afin d'obtenir une fraction d'ARN dépourvue d'ARNr, dans lequel ladite fraction est présente dans une quantité d'au moins 30 ng ;
b) rétrotranscription de la fraction d'ARN dépourvue d'ARNr en ADNc ;
c) purification des ADNc synthétisés ;
d) ligature et amplification des ADNc purifiés afin d'obtenir une banque d'ADNc d'au moins 30 µg,
dans lequel l'étape de rétrotranscription est effectuée en présence d'amorces phosphorylées en 5' ayant l'une des séquences suivantes :
5'-TCGCGATCGTCGNNNNNNNN-3' (SEQ ID N°2) ou 5'- GCGGCCGCNNNNNN-3' (SEQ ID N°1),
dans lesquelles N est l'un quelconque des quatre nucléotides A, T, G et C, et les amorces sont présentes en quantités équimolaires, et dans lequel l'étape d'amplification est effectuée en présence d'ADN polymérase Phi29 ayant une forte activité de déplacement de brin et de relecture, une forte processivité et de forts rendements.

2. Procédé selon la revendication 1, comprenant une étape préliminaire d'extraction de l'ARN total à partir d'un échantillon.

3. Procédé selon l'une quelconque des revendications précédentes, dans lequel les amorces sont présentes dans une quantité d'environ 25 à environ 50 µM et l'ARN dépourvu d'ARNr est présent dans une quantité d'environ 30 ng à environ 300 ng.

4. Procédé selon la revendication 3, dans lequel les amorces sont présentes dans une quantité d'environ 50 µM.

5. Utilisation de la banque d'ADNc spécifique au brin et représentative selon l'une quelconque des revendications 1 à 4 pour un séquençage à haut débit.


### SEQUENCE LISTING

<110> CONSIGLIO NAZIONALE DELLE RICERCHE
   TULLO, Apollonia
   SBISA', Elisabella
   MANGIULLI, Marina
   PESOLE, Graziano
<120> METHOD FOR THE PREPARATION AND AMPLIFICATION OF REPRESENTATIVE AND STRAND-SPECIFIC LIBRARIES OF cDNA FOR HIGH THROUGHPUT SEQUENCING, USE THEREOF, KIT AND CARTRIDGES FOR AUTOMATION KIT
<130> PCT 113779
<150> RM2010A000293
   <151> 2010-05-31
<160> 2
<170> PatentIn version 3.5
<210> 1
   <211> 14
   <212> DNA
   <213> Artificial
<220>
   <223> synthetic primer
<220>
   <221> misc_feature
   <222> (9)..(14)
   <223> n is a, c, g, or t
<400> 1
   gcggccgcnn nnnn 14
<210> 2
   <211> 20
   <212> DNA
   <213> artificial
<220>
   <223> synthetic primer
<220>
   <221> misc_feature
   <222> (13)..(20)
   <223> n is a, c, g, or t
<400> 2
   tcgcgatcgt cgnnnnnnnn 20